# EUROPEAN PATENT SPECIFICATION

(11) **EP 1 218 011 B1**
(45) Date of publication and mention of the grant of the patent: **10.01.2007**
(21) Application number: 00973473.2
(22) Date of filing: 04.10.2000
(51) Int. Cl.: A61K 31/555, A61P 31/12, A61P 31/18, A61P 31/04

(54) **GALLIUM COMPLEXES OF 3-HYDROXY-4-PYRONES TO TREAT INFECTION BY INTRACELLULAR PROKARYOTES, DNA VIRUSES AND RETROVIRUSES**
GALLIUM KOMPLEXE VON 3-HYDROXY-4-PYRONEN ZUR BEHANDLUNG VON DURCH INTRAZELLULÄRE PROKARYOTEN, DNS- UND RETRO-VIREN VERURSACHTEN INFEKTIONEN
COMPLEXES DE GALLIUM DE 3-HYDROXY-4-PYRONES DESTINES AU TRAITEMENT D'INFECTIONS PAR DES PROCARYOTES INTRACELLULAIRES, DES VIRUS A ADN ET DES RETROVIRUS

(30) Priority: 04.10.1999 US 157460 P
(43) Date of publication of application: 03.07.2002
(73) Proprietor: Bernstein, Lawrence R., Menlo Park, CA 94025-2711 (US)
(72) Inventor: Bernstein, Lawrence R., Menlo Park, CA 94025-2711 (US)
(74) Representative: Audier, Philippe André
(86) International application number: PCT/US2000/028174
(87) International publication number: WO 2001/024799

(56) References cited:
- WO-A-93/09776
- WO-A-98/04263
- WO-A-98/09622
- US-A- 5 258 376
- US-A- 5 525 598
- KRATZ F ET AL: "SYNTHESIS AND CHARACTERIZATION OF POTENTIAL ANTITUMOUR AND ANTIVIRAL GALLIUM-III COMPLEXES OF N HETEROCYCLES" POLYHEDRON, vol. 11, no. 4, 1992, pages 487-498, XP002284416 ISSN: 0277-5387
- MAI-LY RAMIREZ ET AL.: "An approach to Substitued 4-hydroxypyran-2-ones" J. HETEROCYCLIC CHEM., vol. 32, 1995, pages 1657-1660, XP002284417

## Description

### TECHNICAL FIELD

The present invention relates generally to the treatment or prevention of intracellular microbial infections, including viral infections. More particularly, the invention relates to the use of certain gallium complexes to manufacture a medicament for the treatment or prevention of infections by intracellular prokaryotes, DNA viruses, including hepatitis B, the papillomavirus family and the herpesvirus family, and retroviruses, including retroviruses causing neoplasms and acquired immunodeficiency syndrome (AIDS) such as the human immunodeficiency virus (HIV) family, and related leukemia and sarcoma retroviruses. Specifically the medicament according to the invention comprises gallium complexes of 3-hydroxy-4-pyrones, including tris(3-hydroxy-2-methyl-4*H*-pyran-4-onato)gallium, also called gallium maltolate.

### BACKGROUND ART

Gallium has shown therapeutic activity in metabolic bone disease, hypercalcemia and cancer (Bernstein (1998), "Mechanisms of therapeutic activity for gallium," *Pharmacol Rev.* 50:665-682). It is approved for use in the United States, as citrate-chelated gallium nitrate solution for intravenous infusion, to treat hypercalcemia of malignancy (Warrell (1995), "Gallium for treatment of bone disease," *Handbook of Metal-Ligand Interactions in Biological Fluids, Bioinorganic Medicine,* 2:1253-1265, New York: Marcel Dekker). Numerous clinical studies have found gallium to have antineoplastic activity, particularly in some lymphomas (Foster et al. (1986), "Gallium nitrate: the second metal with clinical activity," *Cancer Treat Rep* 70:1311-1319), urothelial carcinoma (Einhorn et al. (1994), "Phase II trial of vinblastine, ifosfamide, and gallium combination chemotherapy in metastatic urothelial carcinoma," *J Clin Oncol* 12:2271-2276), and nonsquamous cell cervical carcinoma (Malfetano et al. (1995), "A Phase II trial of gallium nitrate (NSC #15200) in nonsquamous cell carcinoma of the cervix," *Am J Clin Oncol* 18:495-497). The antiproliferative properties of gallium extend to some micro-organisms, and gallium has been suggested as a potential antibiotic, particularly for some intracellular infections such as tuberculosis (Olakanmi et al. (1997), "Gallium inhibits growth of pathogenic mycobacteria in human macrophages by disruption of bacterial iron metabolism: a new therapy for tuberculosis and mycobacterium avium complex?," *J Invest Med* 45:234A). The therapeutic activities of gallium and their proposed mechanisms are discussed by Bernstein (1998), *supra.* The mechanism can be summarized as interfering with cellular uptake of transferrin-bound iron by gallium displacement, inhibiting ribonucleotide reductase, and likely by substitution of iron by gallium in the M2 site of the enzyme ribonucleotide reductase (Bernstein (1998), *supra*).

Without in any way restricting the scope of this invention, it is thought that a primary mechanism for the antineoplastic and general antiproliferative activities of gallium is its ability to substitute for ferric iron in the iron transport protein transferrin (Tf), thereby reducing iron uptake into cells via the transferrin receptor. Evidence of this mechanism is provided by observation that HL60 cells that develop resistance to the antiproliferative action of Ga are also resistant to similar effects of the iron chelating agent deferoxamine and to the effect of monoclonal antibody blockade of the cell Tf receptor (functioning to uptake the iron into the cell) (Chitambar et al. (1991), "Targeting iron-dependent DNA synthesis with gallium and transferrin-gallium." *Pathobiology* 59(1):3-10). Ribonucleotide reductase is an iron-bearing enzyme required for the synthesis of deoxyribonucleotides that are required for the synthesis of DNA. and thus for cell division. Ribonucleotide reductase activity that can be affected by intracellular levels of both iron and gallium affects the life and replication cycles of obligate intracellular prokaryotes, such as chlamydia and rickettsia, DNA viruses and viruses utilizing reverse transcriptase, commonly known as retroviruses. Proliferating cells, due to the enhanced need for ribonucleotide reductase, have a high requirement for iron. Most of the available iron in blood is bound to the iron transport protein Tf, which is also the predominant carrier of gallium in blood plasma. Due to their high iron requirements, proliferating cells overexpress Tf receptor, and therefore take in large amounts of metal-bearing Tf. If gallium is present on the Tf, it will be avidly taken into proliferating cells, thus depleting intracellular iron, and may be incorporated into the M2 site of ribonucleotide reductase. Orally administered gallium, particularly gallium maltolate, has been shown to result in higher Tf binding of absorbed gallium and therefore better tissue distribution than intravenous gallium nitrate (Bernstein (1998), *supra,* Bernstein (2000), "Chemistry and pharmokinetics of gallium maltolate, a compound with high oral gallium bioavailability," *Metal-Based Drugs* 7(1):33-47). Another advantage of the oral gallium over the IV administered gallium nitrate is that no renotoxicity or nephrotoxicity has been observed with oral gallium maltolate (Bernstein (1998), *supra*; Bernstein (2000), *supra*).

Depletion of the iron from the iron-containing M2 site of ribonucleotide reductase, with or without substitution of gallium renders the ribonucleotide reductase non-functional. This in turn depletes the levels of deoxyribonucleotides and diminishes the capacity for production of DNA, in part by depletion of the deoxyribonucleotide reactant and at least in part by organismal (the term "organism" including viruses) regulatory mechanisms that block the initiation of replication. Electron Spin Resonance (ESR) spectra exhibited a markedly reduced ribonucleotide reductase iron signal in cell cytoplasmic extracts from gallium treated HL 60 cells, but the spectra and signal intensity were restored to normal by addition of iron (Chitambar et al. (1991), "Targeting iron-dependent DNA synthesis with gallium and transferrin-gallium," *Pathobiology* 59(1):3-10). Cells unable to produce DNA cannot replicate, and may ultimately undergo apoptosis.

In a similar way, gallium will also prevent the replication of intracellular prokaryotes, DNA viruses and retroviruses, which also must manufacture DNA at some point in their life cycle, and are either directly or indirectly dependent on the host cells' cytoplasmic iron levels for ribonucleotide reductase activity. Some viruses utilize the host's ribonucleotide reductase to produce the deoxyribonucleotides that are major required constituents of DNA. Retroviruses, which must first synthesize DNA from RNA (using the enzyme reverse transcriptase) before new viral particles can be generated, may be particularly sensitive, as they utilize the host's ribonucleotide reductase in the first step of their life cycle within the host cell. Even more complex DNA viruses, such as herpesvirus family members that carry their own ribonucleotide reductase are affected by the intracytoplasmic environment created by the interference with the host cell's iron metabolism.

Many of the current drugs used to treat HIV infection (such as AZT, ddI, ddC) are nucleoside analogs, which inhibit polymerization of DNA as it is replicated; the DNA so formed is prematurely terminated and is non-functional. Gallium is expected to work synergistically with these nucleoside analogs: by inhibiting ribonucleotide reductase, and thus the production of the nucleosides required for DNA synthesis, the relative proportion of nucleoside analogs to native nucleosides will increase, further inhibiting DNA synthesis. Generally, the inhibition of an enzyme combined with the depletion of its substrate are appreciated to be synergistic in terms of reducing production rate of the product. Stapleton et al. (1999), "Gallium nitrate: a potent inhibitor of HIV-1 infection in vitro," *Program and Abstracts, 39^{th} ICAAC Meeting. San Francisco, 1999,* pp. 74, demonstrated the efficacy of gallium nitrate alone to inhibit HIV replication *in vitro* at IC50 concentrations of 4 to 10 µM. They also showed that at subinhibitory concentrations, as expected, gallium nitrate potentiated the inhibitory effects of zidovudine, azidothymidine (AZT), dideoxy inosine (ddI), and dideoxy cytosine (ddC). Both combinations of nucleoside analogs and combinations of nucleoside analogs with non-nucleoside-analog reverse transcriptase inhibitors have been previously demonstrated to be synergistic (Daluge et al. (1997), "152U89, a novel carbocyclic nucleoside analog with potent selective anti-human immunodeficiency virus activity," *Antimicrob. Agents Chemother.* 41(5):1082-93).

Antiretroviral antimicrobials that are active at a different phase of the microbe life cycle than DNA polymerization (such as HIV protease inhibitors), are appreciated to be synergistic in combination with nucleoside analogs that affect DNA synthesis, as has been demonstrated by the of synergistic effects obtained by combining nucleoside analogs with protease inhibitors for retroviral treatment (Daluge et al. (1997), *supra;* Drusano et al. (1998), "Nucleoside analog 1592U89 and human immunodeficiency virus protease inhibitor are synergistic in vitro," *Antimicrob. Agents Chemother.* 42(9):2153-9). Similarly, Poppe et al. (1997), "Antiviral activity of the dihydropyrone PNU-140690, a new noneptidic human immunodeviciency virus protease inhibitor," *Antimicrob. Agents Chemother.* 41(5):1058-63, have shown that nonpeptidic protease inhibitors, structurally distinct from the substrate analog protease inhibitors, are synergistic in combination. Protease inhibitors are specific to treating retroviruses, and only inhibit the protease of the specific virus, thus an HIV I protease inhibitor will not have an equal effect on HIV 2, and may exert no effect on other retroviruses. Such agents, which disrupt a life cycle phase other than DNA replication by targeting a different protein than the DNA polymerase, such as HIV reverse transciptase, are therefore expected to be synergistic with a combination of agents, such as gallium plus a nucleoside analog that are synergistic in inhibiting DNA synthesis by inhibition of reverse transcriptase.

Furthermore, peptides and non-macromolecular hormonal, humoral or hormone-like biomolecules (such as interferons, leukotrienes, interleukins and the like) that stimulate the immune response, particularly the cellular immune response, exert a synergistic effect when combined with anti-microbial agents effective in halting or inhibiting replication of intracellular microbes. This has been demonstrated for a DNA virus by Taylor et al. (1998), "Combined effects of interferon-alpha and acyclovir on herpes simplex type 1 DNA polymerase and alkaline DNase," *Antiviral Res.* 38(2):95-106.

Combination therapy for retroviruses differs from therapy for other viruses in that in addition to the availability of nucleoside analogs and other inhibitors of DNA replication, and hormonal or humoral biological agents that stimulate the immune system, protease inhibitors are retrovirus specific. These agents are currently available for HIV, and are expected to become available for the treatment of other retroviruses such as human T cell leukemia virus (HTLV). An even greater synergistic effect is therefore expected from the combination with a cocktail of anti-virals effective in halting or inhibiting the viral life cycle by a synergistic combination of chemo-inhibition of DNA replication, chemo-disruption of some other phase of the viral life cycle, and hormonal or humoral stimulation of the immune system. For combination therapy of retroviral (HIV 1, HIV 2) or other viral disease, such as Epstein-Barr virus, that compromises the immune system, incorporation of a hormonal or humoral biological agent such as interferon requires that the immune system be sufficiently intact or reconstituted, as by combination chemo-antiviral therapy to mount a specific immune response when stimulated. That is, for the immune stimulating agent to be capable of exerting a synergistic effect, the immune system must be capable of mounting a response, a condition that will only exist early in HIV infection or after a reconstitution of specific cell-mediated immune function by aggressive antiretroviral therapy. Thus, it is not surprising that such synergy has been recently demonstrated for patients with sufficiently high CD4+ cell counts (Losso et al. (2000), "A randomized, controlled, phase 11 trial comparing escalating doses of subcutaneous interleukin-2 plus antiretrovirals versus antiretrovirals alone in human immunodeficiency virus-infected patients with CD4+ cell counts >/= 350/mm³," *J Infect. Dis.* 181(5):1614-21). The recent addition of protease inhibitors to the combination therapy regime has allowed restoration of HIV specific immune response, a reconstitution or restoration of the immune system that had been predicted for early HIV disease treated with highly active antiretroviral treatment (HAART) (Al-Harthi et al. (2000), "Maximum suppression of HIV replication leads to the restoration of HIV-specific responses in early HIV disease," *AIDS* 14(7):761-70), leading to the expectation that immune stimulating hormono-humoral biomolecules, such as leukotrienes and interferons, will become useful additions to the routine treatment of HIV disease.

Oral gallium is another immune system independent agent that can both bolster antiretroviral therapy and be used against other pathogens, such as DNA viruses. An acknowledged advantage of combination therapy is that it reduces the emergence of resistant strains because of the low probability of a single organism simultaneously acquiring multiple mutations conferring resistance (Drusano (1998), *supra).* The greater the structural and mechanistic differences between the combined agents, the more protection there is against simultaneous multiple resistance-conferring mutations because of the distance between the genetic loci, as when the agents target different molecular targets (Drusano (1998), *supra).* As the mechanism of gallium action is by disruption of the host cell iron uptake metabolism, to affect the levels of deoxyribonucleotide substrate for the DNA polymerase by affecting the iron-bearing site of ribonucleotide reductase, the addition or substitution of gallium to existing combination therapy antiviral regimens increases the likelihood that emergence of resistant viruses can be delayed or prevented. Further, as the mechanism of gallium action is to a great extent dependent on the somatic host cell, which is not evolving, gallium by virtue of the aforementioned mechanism is inherently less likely to support development of resistance to it than agents that act directly against viral proteins such as protease inhibitors and nucleoside analogs.

Gallium has been shown *in vitro* to inhibit the enzyme reverse transcriptase in Rauscher murine leukemia virus (Waalkes et al. (1974), "DNA polymerases of Walker 256 carcinoma," *Cancer Res* 34:385-391). As this murine retrovirus is related to HIV, this mechanism would likely operate on HIV and other related human retroviruses. Moreover, reverse transcriptase is appreciated to be an RNA-dependent DNA polymerase which, like all DNA polymerases, requires deoxyribonucleotides supplied by an active ribonucleotide reductase. Thus, it expected that any viral or non-viral intracellular microbe that uses a DNA polymerase, and therefore requires deoxyribonucleotides as substrate, will be susceptible to the iron depletion and gallium enrichment ultimately effected by circulating Tf-bound gallium in the host cell's cytoplasm. This is expected even when the organism has its own ribonucleotide reductase and DNA polymerase, as do members of the herpesvirus family. Additionally, as in the case of intracellular prokaryotes, the microbe has its own protoplasm comprising cytoplasm and nucleoid, because the protoplasm is expected to take on the iron depleted and gallium enriched attributes of the host cell's cytoplasm.

An orally active gallium compound was sought as a more convenient, comfortable, safe, and less costly alternative to parenterally administered gallium; in addition, such a compound could be used for daily administration to chronically ill patients. Such a compound could be administered to already immuno-compromised HIV infected individuals to treat or prevent opportunistic infections by susceptible infectious agents, including systemic HHV-1 (HSV1), HHV-2 (HSV2), HHV-3 (VZV), HHV-4 (EBV), HHV-5 (CMV), HHV-7, HHV-8 (KSV) and retinitis caused by CMV (HHV-4) or another herpesvirus and, for chemo-prevention of common virally caused neoplasms of AIDS, such as Kaposi Sarcoma, now believed to be caused by HHV-8 (KSV), and sometimes HHV-7 or HHV-7 with HHV-6, lymphomas caused by HHV-4 (EBV), HHV-8 (KSV), and sometimes HHV-7 or HHV-7 with HHV-6.

Gallium is absorbed very poorly when orally administered as salts such as the chloride or nitrate (Collery et al. (1989), "Clinical pharmacology of gallium chloride after oral administration in lung cancer patients," *Anticancer Res.* 9:353-356; Ho et al. (1990), "Bioavailability of gallium nitrate," *Eur. J. Pharmacol.* 183:1200), due in part to hydrolysis that produces low-solubility polymerized gallium oxide hydroxides in the gastrointestinal fluids. In animal and clinical studies, gallium maltolate, tris(3-hydroxy-2-methyl-4*H*-pyran-4-onato)gallium (GaM), is found to provide oral gallium absorption roughly ten times higher than from gallium salts.

Gallium maltolate is a coordination complex of a trivalent gallium ion with three deprotonated maltol (maltolate) groups. Maltol (2-methyl-3-hydroxy-4*H*-pyran-4-one) is produced by some plants and is commonly formed when sugars are heated: it is largely responsible for the scent of cotton candy and contributes significantly to the fragrance of many cakes, cookies, and candies. Its ability to provide a "fresh-baked" fragrance and to enhance sweet flavors has led to its extensive use as a food additive (LeBlanc et al. (1989), "Maltol and ethyl maltol: from the larch tree to successful food additive," *Food Technology* 43:78-84).

Methods to synthesize gallium complexes of 3-hydroxy-4-pyrones, the preparation of such complexes in pharmaceutical formulations, and several methods for their use in pharmaceutical applications have been presented by Bernstein; see U.S. Patent Nos. 5,258,376, 5,574,027, 5,883,088, 5,968,922, 5,981,518, 5,998,397, 6,004,951, 6,048,851 and 6,087,354.

The use of gallium complexes of 3-hydroxy-4-pyrones to treat intracellular prokaryote and viral infections is, to date, unknown. The use of these complexes to treat multiple or co-infections by DNA viruses, retroviruses and intracellular prokaryotes is also unknown. The present invention is premised on the important finding that these complexes, including gallium maltolate, are exceptionally effective at treating obligate intracellular prokaryotes, including mycoplasma, rickettsia, and chlamydia, DNA viruses, including adenovirus, hepatitis B, herpesvirus family (human and non-human) and retroviral infections, including HIV and HTLV, particularly in combination with other antiretroviral drugs. Further, these complexes, including gallium maltolate, are exceptionally effective at treating the pathogens listed above in immunocompromised HIV infected individuals. Even certain eukaryotic parasites that replicate their genome intracellularly are susceptible to the broad mechanism of gallium action. Non-obligate intracellular prokaryotes such as macrophage phagocytosed bacteria that are not easily killed once internalized by the macrophage, such as *Mycobacterium tuberculosis, Mycobacterium leprae, Mycobacterium avium* and other mycobacteria species, are also susceptible to the Tf-bound gallium, Tf-receptor mediated mechanism of action of orally administrable gallium compounds. As the phagocytosed individuals of such phagocytosis resistant organisms render an infection difficult to treat, the gallium compounds of the instant invention can also find use in combination treatments with agents that are more effective against non-phagocytosed members of the infecting population.

Also, it has been discovered that gallium maltolate and related gallium complexes of hydroxypyrones provide a safe and effective way to administer gallium orally to patients with the described infections and co-infections. Because of the synergistic mechanism of the compounds of the instant invention with respect to other retroviral agents, as well as with nucleoside analogs and immune-stimulating biomolecules against DNA viruses and bacteriostatic agents useful against prokaryotes, it is expected to be especially useful against co-infections by the specified agents in general and indispensible in treating opportunistic or refractory infections in immunocompromised HIV patients.

### DISCLOSURE OF THE INVENTION

Accordingly, it is a primary objective of the invention to address the above-mentioned need in the art by providing the use of certain gallium complexes to manufacture pharmaceutical composition for treating or preventing obligate intracellular prokaryote, DNA virus and retroviral infections. These composition comprise gallium complexes of 3-hydroxy-4-pyrones, particularly gallium maltolate, and can be administered to humans and other mammalian subjects who have obligate intracellular prokaryote, DNA virus or retroviral infections or who may have been exposed to these infectious agents and have need to prevent infection.

A secondary objective of the invention is to address the specific need for agents that can be synergistically combined with regimes against different co-infecting susceptible microbes. Most importantly, for HIV-infected individuals, the objective is to provide a pharmacologic agent that can bolster their anti-HIV regimen while simultaneously having an effect against common non-opportunistic co-infective agents such as hepatitis B and hepatitis C, and against the opportunistic infections that primarily cause the morbidity and mortality in immunocompromised HIV patients, including the herpesvirus family members that often opportunistically emerge from latency to debilitate and ultimately kill the patient either by direct viral infection or by inducing a neoplasm. Additional objects, advantages and novel features of the invention will be set forth in part in the description that follows, and in part will become apparent to those skilled in the art upon examination of the following, or may be learned by practice of the invention.

### MODES FOR CARRYING OUT THE INVENTION

As noted above, the present invention is directed to the use of certain gallium complexes to manufacture a medicament for treating and preventing retroviral infections using gallium complexes of 3-hydroxy-4-pyrones. Prior to discussing this invention in further detail, the following terms will first be defined. Unless defined below, the terms used herein have their normally accepted meanings.

### 1. DEFINITIONS:

As used herein, the following terms have the definitions given below:

The term "neutral 3:1 gallium complex of a 3-hydroxy-4-pyrone" refers to an electrostatically neutral complex of Ga³⁺ and 3 equivalents of the anionic form of a 3-hydroxy-4-pyrone which complex is represented by the formula [Ga³⁺(py⁻)₃] wherein py⁻ represents the anionic form of a 3-hydroxy-4-pyrone as defined below. Because such complexes do not dissociate to any significant extent in aqueous solutions maintained at a pH of from about 5 to about 9, these complexes remain predominantly electrostatically neutral in such solutions.

In this regard, these complexes are deemed "electrostatically neutral" because there are equal numbers of positive and negative charges in the complex.

Also, it is apparent that the anionic form of the 3-hydroxy-4-pyrone acts as a chelating agent to the gallium and as such, the complex is sometimes referred to herein as a "neutral gallium chelate of a 3-hydroxy-4-pyrone," it being understood that this latter term is synonymous with the term "neutral 3:1 gallium complex of a 3-hydroxy-4-pyrone."

The term "a 3-hydroxy-4-pyrone" refers to a compound of Formula 1: 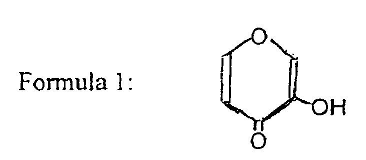 wherein from zero to three of the hydrogen atoms attached to the ring carbon atoms are replaced by a hydrocarbon group of from one through six carbon atoms.

Specific compounds encompassed by the term "a 3-hydroxy-4-pyrone" are represented by the Formulas 2-5 below: 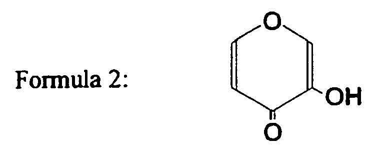 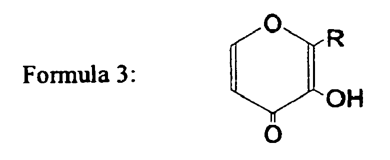 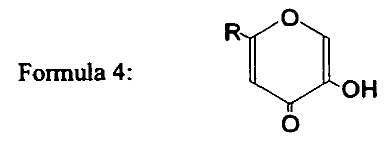 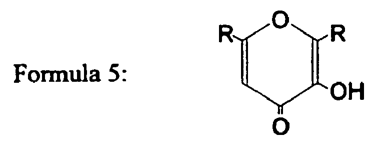 wherein each R is independently a hydrocarbon of from 1 to 6 carbon atoms.

The unsubstituted form of 3-hydroacy-4-pyrone (Formula 2, also called pyromeconic acid) contains three hydrogen atoms that are bound only to ring carbon atoms. As noted above, any combination of these three hydrogen atoms can be substituted with a hydrocarbon group and all possible combinations of such substitutions are encompassed within this invention. The locations of a few possible substitutions are presented in Formulae 3-5, in which R is a hydrocarbon group (including methyl, ethyl, isopropyl, and n-propyl). The hydrocarbon groups are preferably acyclic and are preferably unbranched. Groups containing six or fewer carbon atoms, particularly of one through three carbon atoms, especially methyl or ethyl, are preferred. Single substitution is preferred; a substitution at either the 6-position or especially the 2-position is preferred. Some examples of specific compounds whose gallium complexes may be used in compositions herein are: 3-hydroxy-2-methyl-4-pyrone (Formula 3, R=CH₃ ; sometimes referred to as maltol or larixinic acid) and 3-hydroxy-2-ethyl-4-pyrone (Formula 3, R=C₂H₅ ; sometimes referred to as ethyl maltol or ethylpyromeconic acid), both of which are preferred for use in this invention, especially 3-hydroxy-2-methyl-4-pyrone. Other preferred compounds include 3-hydroxy-4-pyrone (Formula 2; sometimes referred to as pyromeconic acid) and 3-hydroxy-6-methyl-4-pyrone (Formula 4, R=CH₃).

The term "an anion of a 3-hydroxy-4-pyrone" refers to a compound defined in Formulae 2-5 above wherein the hydroxyl proton has been removed so as to provide for the anionically charged form of the compound.

The terms "oral administration" and "oral ingestion" refer to all conventional forms for the oral delivery of a pharmaceutical composition to a patient (e.g., human) and that result in the deposition of the pharmaceutical composition into the gastrointestinal tract (including the gastric portion of the gastrointestinal tract, i.e., the stomach) of the patient. Accordingly, oral administration and oral ingestion include, by way of example, actual ingestion of a solid or liquid pharmaceutical composition, oral gavage, and the like.

The term "inhibit dissociation" means that at least 20%, preferably at least 50% and more preferably at least 80%, of the complex is not dissociated under acidic conditions (e.g., about pH 2-4) for a period of at least 0.5 hr and preferably at least 2 hours.

By the term "effective" or "therapeutically effective" amount of a drug is meant a nontoxic but sufficient amount of a compound to provide the desired effect at a reasonable benefit/risk ratio attending any medical treatment. The desired effect may be alleviation of the signs, symptoms, or causes of a disease, or any other desired alteration of a biological system.

The terms "active agent," "pharmacologically active agent" and "drug" are used herein to refer to a complex of a hydroxypyrone and gallium, particularly a neutral 3:1 gallium (III) complex of a 3-hydroxy-4-pyrone.

The term "treat." as in to "treat" a condition, is intended to include (1) preventing the condition. i.e., avoiding any clinical symptoms of the condition, (2) inhibiting the condition, that is, arresting the development or progression of clinical symptoms, and/or (3) relieving the condition. i.e., causing regression of clinical symptoms.

The term "individual" as in treatment of "an individual" is intended to refer to an individual organism afflicted with or prone to a condition, disorder or disease as specified herein, and includes both humans and animals.

By "pharmacologically acceptable" is meant a material that is not biologically or otherwise undesirable, i.e., the material may be administered to an individual along with the active agent without causing any undesirable biological effects or interacting in a deleterious manner with any of the other components of the pharmaceutical composition in which it is contained.

"Optional" or "optionally" means that the subsequently described circumstance may or may not occur, so that the description includes instances where the circumstance occurs and instances where it does not. For example, recitation of an additive as "optionally present" in a formulation herein encompasses both the formulation containing the additive and the formulation not containing the additive.

"Intracellular prokaryote" refers to a prokaryote that is alive within a cell. The term is meant to include "obligate intracellular prokaryote" (defined below), and prokaryotes that can survive and are in fact inside a host cell. Macrophage-phagocytosed *Mycobacterium tuberculosis* and *Mycobacterium leprae* are two examples of intracellular prokaryotes that are not obligate intracellular prokaryotes.

"Obligate intracellular prokaryote" refers to a prokaryote that must live within a host cell. The term "obligate intracellular prokaryote," is meant to encompass those prokaryotes which resemble viruses in that they can not complete their life cycle outside a host cell. By way of example rather than limitation of this definition, prokaryotes that are obligately intracellular include *Mycoplasma, Chlamydia,* and *Rickettsia* species, which are important pathogens.

"DNA virus'' means any virus that carries its genome in the infective viral particle as DNA, and thus must make DNA to replicate. Such viruses comprise the majority of tumor virus species. By way of example rather than limitation of this definition, DNA viruses include the adenovirus, adeno associated virus, papillomavirus, and herpesvirus groups. Specific examples of DNA viruses include hepatitis B virus, SV 40, individual human papillomavirus species and, individual equine, feline, canine, simian, murine, avian and human herpes virus species, which include human herpesvirus 1-8 (HHV-1 - HHV-8). The human herpesviruses are important, often opportunistic, pathogens also known as follows: HHV-1 is Herpes Simplex I (HSV1), HHV-2 is Herpes Simplex II (HSV2), HHV-3 is Herpes Varicella Zoster I (HVZ or VZV), HHV-4 is Epstein-Barr Virus (EBV), HHV-5 is Cytomegalovirus (CMV), HHV-6, HHV-7, and HHV-8 is now being termed Kaposi Sarcoma Associated Virus (KSV).

"Retrovirus," or "retroviral" refer to any virus that carries its genome in the infective viral particle as single stranded RNA (ssRNA) and as part of its replicative cycle makes a DNA provirus from the ssRNA of the infectious particle by use of a unique RNA template dependent DNA Polymerase, known as reverse transcriptase. A taxonomic definition of retrovirus is a virus that belongs to the family *Retroviridae.* By way of example, retroviruses include the human spumavirus, Mason-Pfizer monkey bovine leukaemia virus, mouse mammary tumor virus, avian leukosis virus, murine leukemia virus, rous sarcoma virus, feline leukemia virus (FELV), feline immunodeficiency virus (FIV), simian immunodeficiency virus (SIV), hepatitis C virus, human T cell leukemia species (HTLV1, 2), HIV-1, HIV-2. Also expressly included in the definition of retrovirus are endogenous retroviruses, which are known to comprise approximately 1-2 percent of the genomes of animal species. These endogenous retroviruses are normally latent and non-pathogenic to the species to which they are endogenous, and may be pathogenic to their native species. Human endogenous retroviruses (HERV) include those shown to be derived from feline murine equine and other retroviruses from species which have been in contact with humans through evolution. Different mechanisms including xenograft transplantation with exposure of the xenograft to pathogenic tumor viruses, infection with other tumor viruses including HIV, and transposition can cause the ERV to evolve to become a pathogen to the species in which it was endogenous or to another species.

"HIV infection" and "infection by HIV" refer to infection by one or more members of the group of retroviruses that are members of the primate lentivirus group of the genus *Lentiviridae* and are capable of infecting a human whether or not this capability has been demonstrated. HIV-1 and HIV-2 are examples of primate lentiviruses that are known to infect humans. HIV-1 infection, HIV-2 infection or infection by both HIV-1 and HIV-2 are within this definition. Infection of a human by a lentivirus that is not named and differs from all known HIV strains is also contemplated as within this definition.

It must be noted that as used herein and in the claims, the singular forms "a," "and" and "the" include plural referents unless the context clearly dictates otherwise. Thus, for example, reference to "an active agent" in a formulation includes two or more active agents, reference to "a carrier" includes two or more carriers, and so forth.

### 2. SYNTHESIS AND METHODOLOGY:

The 3:1 gallium complexes of 3-hydroxy-4-pyrone or 3-hydroxy-4-pyrones useful herein may be synthesized by reacting such hydroxypyrones with gallium ions and isolating, at least in part, the resulting complex or complexes.

Specifically, the neutral 3:1 gallium complex of a 3-hydroxy-4-pyrone is prepared by the reaction of gallium ions and the 3-hydroxy-4-pyrones in solution. Gallium ions can be derived from a gallium salt, such as a gallium halide, particularly gallium chloride, or a gallium nitrate compound, especially a hydrated gallium nitrate. The gallium nitrate compounds are often preferable as they are easier to work with than gallium halides, which may be highly irritating and may react violently with many solvents, including water. With the proper safeguards, a variety of gallium salts can be used. The reaction is conveniently effected in a mutual solvent, including but not limited to mixtures containing water, ethanol, methanol, and chloroform. Pure water may be used in many cases. A preferable method, if it is desired to separate at least a major part of reaction by-products such as sodium nitrates, sodium chloride, and sodium carbonates, is to use a mixture containing roughly equal parts of ethanol and chloroform, with a trace of water. The reaction by-products mentioned above have very low solubilities in this mixture and can be removed readily by filtration.

To produce the preferred neutral 3:1 hydroxypyrone:gallium complex, the hydroxypyrone and the gallium ions are mixed in 3:1 molar proportions, preferably with a slight excess of hydroxypyrone to insure a great preponderance of the 3:1 complex over the 2:1 and 1:1 complexes. The proportions of the particular complexes formed are dependent upon the pH of the solution. When a gallium salt such as a halide or nitrate is dissolved, the resulting solution will generally have a low pH. To form a preponderance of the preferred neutral 3:1 complex, a pH of from 5 to 9, preferably 7 through 8, is used. If a more acidic solution is used, a preponderance of the less preferred 2:1 and 1:1 complexes may instead be formed, even if a large excess of hydroxypyrone is present. Under highly basic conditions, poorly soluble gallium hydroxides may precipitate. It is preferable to regulate the pH with materials other than hydroxides such as sodium hydroxide, as the use of such hydroxides may cause the precipitation of poorly soluble gallium hydroxides, which are not wanted, and the pH may actually be buffered at an undesirable level by the precipitate. The use of a carbonate, especially sodium carbonate, is preferred to regulate the pH. The use of sodium carbonate in a solvent mixture containing ethanol and chloroform, for example, can result in the precipitation of sodium nitrates that are very slightly soluble in this mixture, and which can be filtered off if desired to help purify the solution containing the desired pharmaceutical compositions.

The reaction to form the hydroxypyrone-gallium complex in solution is generally complete within about five minutes at about 20° C. Gentle stirring or other agitation of the solution promotes a uniform, rapid reaction. Longer reaction times may be used as necessary. If desired, following the separation of reaction by-products such as sodium nitrates, sodium chloride, and sodium carbonates (depending an the solvents and reactants used), the reaction mixture may be evaporated slowly in air or, more rapidly, through the use of a rotary evaporator or by freeze drying, as examples. After drying, the gallium complex or complexes will remain in solid form. Recrystallization can be accomplished, if desired, using a suitable solvent, including but not limited to chloroform, alcohols such as ethanol and methanol, ether, water, acetone, and mixtures containing such solvents. Suitable solvents will depend upon which particular gallium complex(es)and impurities are present, upon the impurities to be separated, and upon the temperature and other physical conditions.

It is noted that the mentioned methods are not the only ones that can produce hydroxypyrones and gallium complexes with hydroxypyrones and that various alternative methods may be used as will be apparent to those skilled in the art. Additionally, in preparing the neutral 3:1 complexes of gallium with 3-hydroxy-4- pyrone, a single 3-hydroxy-4-pyrone or a mixture of 3-hydroxy-4-pyrones can be used. However, preferably, only a single 3-hydroxy-4-pyrone is employed.

With regard to the preparation of 3-hydroxy-4-pyrones that are used as starting materials in the preparation of the neutral 3:1 complexes of gallium with 3-hydroxy-4-pyrones, certain of these compounds occur naturally and may be obtained by extraction from the natural sources. For example, maltol is found in the bark of the young larch tree (*Larix decidua Mill.*), and in pine needles, chicory, wood tars and oils, and roasted malt (*Merck Index,* 9^{th} Edition, pp. 741-742, Rahway, NJ: Merck & Co., 1976). Certain of the 3-hydroxy-4-pyrones are available commercially, including maltol and ethyl maltol. Others can be made from pyromeconic acid as a starting material, which can be derived from the decarboxylation of meconic acid. Methods for preparing such other 3-hydroxy-4-pyrones are well known in the art. Additionally, it is noted that maltol and ethyl maltol are in widespread use as flavoring and fragrance-enhancing agents for foods, and have very low toxicities when taken orally.

### 3. PHARMACEUTICAL COMPOSITIONS:

The methods of this invention are achieved by using a pharmaceutical composition comprising a neutral 3:1 complex of gallium with 3-hydroxy-4-pyrone. The compounds may be administered orally, parenterally (including by subcutaneous, intravenous and intramuscular injection), transdermally, rectally, nasally, buccally, sublingually, topically, vaginally, etc., in dosage formulations containing one or more conventional non-toxic pharmaceutically acceptable carriers.

Depending on the intended mode of administration, the pharmaceutical compositions may be in the form of solid, semi-solid or liquid dosage forms, such as, for example, tablets, suppositories, pills, capsules, powders, liquids, suspensions, creams, ointments, lotions or the like, preferably in unit dosage form suitable for single administration of a precise dosage. The compositions contain an effective amount of the active agent, generally although not necessarily in combination with a pharmaceutically acceptable carrier and, in addition, may include other pharmaceutical agents, adjuvants, diluents, buffers, etc.

For solid compositions, conventional nontoxic carriers include, for example, pharmaceutical grades of mannitol, lactose, starch, magnesium stearate, sodium saccharin, talc, cellulose, glucose, sucrose, magnesium carbonate, and the like. Liquid pharmaceutically administrable compositions can, for example, be prepared by dissolving, dispersing, etc., an active agent as described herein and optional pharmaceutical adjuvants in an excipient, such as, for example, water, saline, aqueous dextrose, glycerol, ethanol, or the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example, see *Remington: The Science and Practice of Pharmacy,* 19^{th} Ed., Easton PA: Mack Publishing Co., 1995.

For oral administration, the composition will generally take the form of a tablet or capsule, or may be an aqueous or nonaqueous solution, suspension or syrup. Tablets and capsules are preferred oral administration forms. Tablets and capsules for oral use will generally include one or more commonly used carriers such as lactose and corn starch. Lubricating agents, such as magnesium stearate, are also typically added. When liquid suspensions are used, the active agent may be combined with emulsifying and suspending agents. If desired, flavoring, coloring and/or sweetening agents may be added as well. Other optional components for incorporation into an oral formulation herein include, but are not limited to, preservatives, suspending agents, thickening agents, and the like. Particularly preferred oral formulations herein, as will be described in further detail below, are delayed release formulations such as enteric coated tablets.

Parenteral administration is generally characterized by injection. Injectable formulations can be prepared in conventional forms, either as liquid solutions or suspensions, solid forms suitable for solution or suspension in liquid prior to injection, or as emulsions. Preferably, sterile injectable suspensions are formulated according to techniques known in the art using suitable dispersing or wetting agents and suspending agents. The sterile injectable formulation may also be a sterile injectable solution or a suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium.

The compounds of the invention may also be delivered through the skin or mucosal tissue using conventional "transdermal"-type patches, wherein the agent is contained within a laminated structure that serves as a drug delivery device to be affixed to the skin. In such a structure, the drug composition is contained in a layer, or reservoir, underlying an upper backing layer. The laminated structure may contain a single reservoir, or it may contain multiple reservoirs. In one embodiment, the reservoir comprises a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are present as separate and distinct layers, with the adhesive underlying the reservoir which, in this case, may be either a polymeric matrix as described above, or it may be a liquid or hydrogel reservoir, or may take some other form.

Alternatively, the pharmaceutical compositions of the invention may also be administered in the form of suppositories for rectal administration. These can be prepared by mixing the agent with a suitable non-irritating excipient which is solid at room temperature but liquid at the rectal temperature and therefore will melt in the rectum to release the drug. Such materials include cocoa butter, beeswax and polyethylene glycols.

The pharmaceutical compositions of the invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, propellants such as fluorocarbons or nitrogen, and/or other conventional solubilizing or dispersing agents.

Preferred formulations for topical drug delivery are ointments and creams. Ointments are semisolid preparations which are typically based on petrolatum or other petroleum derivatives. Creams containing the selected active agent, are, as known in the art, viscous liquid or semisolid emulsions, either oil-in-water or water-in-oil. Cream bases are water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. The specific ointment or cream base to be used, as will be appreciated by those skilled in the art, is one that will provide for optimum drug delivery. As with other carriers or vehicles, an ointment base should be inert, stable, nonirritating and nonsensitizing.

As noted above, however, preferred compositions herein are oral formulations, and particularly preferred oral formulations are "delayed release." It has now been discovered that while the neutral 3:1 complex of gallium with 3-hydroxy-4-pyrones delivers gallium to the bloodstream from the gastrointestinal tract, partial dissociation may occur of the neutral 3:1 complex of gallium with 3-hydroxy-4-pyrone under acidic conditions (generally at a pH of about 4 or less). Such acidic conditions may be present in the stomach. The dissociation may result in formation of the less absorbable 2:1 and 1:1 complexes, together with free hydroxypyrone and ionic gallium. Accordingly, in order to maintain the orally delivered gallium in a form that is highly absorbable in the gastrointestinal tract, the pharmaceutical compositions of this invention may be formulated to contain a means to inhibit dissociation of this complex when exposed to the acidic conditions of the stomach.

Means to inhibit or prevent dissociation of this complex when exposed to the acidic conditions of the stomach include the following preferred methods:
(1) Addition of a sufficient amount of a pharmaceutically compatible buffering agent to the 3:1 complex that would bring the pH of the stomach fluids to a pH in the range of approximately 5 to 9, preferably in the range of approximately 6 to 7, so that the stomach fluids would no longer disrupt the 3:1 hydroxypyrone:Ga complex.
   Pharmaceutically compatible buffering agents are those which, while acting as buffering agents, do not significantly alter the ability of the neutral 3:1 gallium complex to deliver gallium to the bloodstream of the patient and are not toxic either alone or in combination with the neutral gallium complex. The particular pharmaceutically compatible buffering agent employed is not critical. Examples of preferred pharmaceutically compatible buffering agents include, by way of example, calcium carbonate (CaCO₃), sodium bicarbonate (NaHCO₃) and the like. On the other hand, aluminum hydroxide, Al(OH)₃, and other aluminum-containing compounds, by way of example, should be avoided. Other pharmaceutically compatible buffering agents are well known in the art and are recited in standard pharmaceutical manufacturing textbooks (e.g., *Remington: The Science and Practice of Pharmacy, supra*).
(2) Adding to the pharmaceutical composition containing the 3:1 complex an excess of free hydroxypyrone (or a salt thereof containing a physiologically acceptable cation), particularly the one used to make the 3:1 complex. Such a mixture, when dissolved in the stomach, has the effect of shifting the equilibrium among the 1:1, 2:1, and 3:1 complexes towards a preponderance of the 3:1 complex. In this embodiment, the weight of the free hydroxypyrone incorporated into the formulation is preferably 0.1 to 100 times the weight of the 3:1 complex employed in the formulation, and more preferably 0.1 to 10 times. This method, by itself, is not highly preferred but may be used in conjunction with other methods to inhibit dissociation.
(3) Formulating the pharmaceutical composition that contains the 3:1 complex in delayed release form, so that a preponderance of the complex is not released until the intestinal tract is reached. An example of such a composition is to formulate the 3:1 complex with certain gels, preferably hydrogels such as a polymerized polyethylene glycol hydrogel, that adsorb the 3:1 complex and then release it after ingestion only very slowly while in the stomach. The preparation of such delayed release formulations, particularly those using hydrogels, is well known in the art.
(4) Most preferably, formulating or packaging the 3:1 complex in such a way that the release of the 3:1 complex is prevented or inhibited until the basic, or less acidic, conditions of the intestinal tract are reached. Specific preferred methods include:
   (a) Encapsulating the 3:1 complex in a material that is resistant to dissolution until the intestinal tract is reached, most preferably using a tablet or capsule that is enteric coated, or granules that are enteric coated, to inhibit or prevent release of the 3:1 complex until a pH greater than about 5 or 6 is reached. Enteric coating of tablets, capsules, and granules is well known in the art.
   (b) Microencapsulating the 3:1 complex within liposomes, preferably made from phospholipids, that do not, dissociate under the acidic conditions of the stomach, but that will release the 3:1 complex in the higher pH conditions of the intestinal tract. Such liposomes are also well known in the art.

The most preferred method, enteric coating tablets, granules or especially capsules, is well known in the art.

Preferred materials for the enteric coating include, by way of example, cellulose butyrate phthalate, cellulose hydrogen phthalate, cellulose proprionate phthalate, polyvinyl acetate phthalate, cellulose acetate phthalate, cellulose acetate trimellitate, hydroxypropyl methylcellulose phthalate, hydroxypropyl methylcellulose acetate, carboxymethyl ethylcellulose, hydroxypropyl methylcellulose acetate succinate, and acrylic acid and acrylic ester polymers and copolymers, preferably formed from acrylic acid, methacrylic acid, methyl acrylate, ethyl acrylate, methyl methacrylate and/or ethyl methacrylate (as commercially available under the tradename Eudragit^{®}), with cellulose acetate phthalate preferred. When capsules are coated, a plasticizer should be used to minimize brittleness in the coating and to inhibit cracking of the coating. Tablets and granules can also be used.

For enteric coated tablets, the core of the coated formulation will generally contain other materials such as binders, diluents, lubricants, disintegrants, fillers, stabilizers, surfactants, coloring agents, and the like. An additional active agent may also be included if desired.

The core of the formulation will generally, although not necessarily, contain approximately 5 to 95 wt.% active agent, with the remainder of the core comprising binders and other materials as described above.

In addition to the above, two or more means to inhibit dissociation of these complexes can be employed in combination so as to enhance the level of inhibition, i.e., a pharmaceutically compatible buffer can be employed in combination with an excess of free 3-hydroxy-4-pyrone.

The preferred formulations for oral administration herein are solid unit dosage forms, e.g., enteric coated tablets as described above, wherein each unit dosage form contains a therapeutically effective amount of active agent. Delayed release capsules may also be used, wherein the active agent, with or without additional hydroxypyrone, buffers, or other active ingredients, may be encapsulated without carriers or the like, as the capsule can itself serve as the means to inhibit dissociation of the complex.

Oral formulations including a liquid pharmaceutically inert carrier (e.g., water) may also be considered for oral administration, wherein the formulations preferably include an appropriate means for inhibiting dissociation of the 3:1 complex in the acidic conditions of the stomach, preferably through the use of a pharmaceutically compatible buffer, preferably CaCO₃ or NaHCO₃. The use of such buffers is well known in the art.

The practice of the present invention will employ, unless otherwise indicated, conventional techniques of drug formulation, which are within the skill of the art. Such techniques are fully explained in the literature: see, as examples, *Remington: The Science and Practice of Pharmacy,* 19^{th} Ed., Easton PA: Mack Publishing Co., 1995, *supra;* and Goodman & Gilman's *The Pharmacological Basis of Therapeutics,* 9^{th} Ed., New York: McGraw-Hill, 1996.

### 4. METHODS OF PHARMACEUTICAL TREATMENT:

One embodiment of this invention involves administering a therapeutically effective amount of gallium to a mammalian individual in the form of a complex of gallium and a hydroxypyrone as defined earlier herein. The complex preferably although not necessarily consists essentially of a neutral 3:1 (hydroxypyrone:gallium) complex in which the hydroxypyrone is either unsubstituted or substituted with one through three C,-C₆ alkyl substituents that may be the same or different, and wherein the therapeutically effective amount is such that a blood plasma gallium concentration is provided that is sufficient to enable treatment or prevention of the obligate intracellular prokaryote, DNA virus or retroviral disease. A highly preferred complex is the 3:1 complex of maltol with gallium (3-hydroxy-2-methyl-4-pyrone): tris(3-hydroxy-2-methyl-4*H*-pyran-4-onato)gallium, also called gallium maltolate. Another preferred complex is the 3:1 complex of gallium with ethyl maltol (3-hydroxy-2-methyl-4-pyrone): tris(3-hydroxy-2-ethyl-4*H*-pyran-4-onato)gallium, also called gallium ethyl maltolate.

In a preferred embodiment of the invention, the complex is administered orally in solid dose form by a tablet or capsule containing one or more pharmaceutically acceptable carriers. Administration may also be orally in liquid dose form together with one or more pharmaceutically acceptable carriers, and also by other means, including parenteral, transdermal, rectal, intranasal, buccal, intraocular, sublingual, topical, topical ocular, vaginal, or through the lung by inhalation.

For oral administration, the therapeutic plasma levels are approximately 1 to 5,000 ng/mL, particularly approximately 200 to 1000 ng/mL. Oral doses to achieve these therapeutic levels are approximately 10 to 2,500 mg of the complex per day, particularly approximately 250 to 750 mg per day. The complex is preferably administered in single dose form, but may be administered in multiple doses per day. The complex is preferably administered at least one hour before meals and at least two hours after meals, but other schedules are also acceptable.

The complex may be administered together with other antiretroviral agents, particularly those used in AIDS therapy, including without limitation nucleoside analogs such as zidovudine (AZT), ddI and ddC, protease inhibitors such as saquinavir, ritonavir, indinavir, and nelfinavir, and non-nucleoside reverse transcriptase inhibitors such as nevirapine and delavirdine. The invention includes formulations that include active agents other than the gallium complex.

Dose schedules to treat patients infected with HIV-1 include, as a few representative examples without in any way limiting the scope of this invention: (a) 500 mg gallium maltolate once per day, plus 200 mg AZT twice per day, plus 200 mg ddI twice per day, plus 800 mg indinavir every eight hours; (b) 400 mg gallium maltolate twice per day, plus 200 mg AZT twice per day, plus 0.75 mg ddC three times per day, plus 600 mg ritonavir twice per day; (c) 250 mg gallium maltolate once per day, plus 40 mg d4T twice per day, plus 200 mg ddI twice per day, plus 750 mg nelfinavir three times per day. These examples are for a 60 kg individual; adjustments for different weights may be made.

Infections that may be treated with the uses of this invention include retroviral and DNA virus infections. Retroviral infections include those associated with AIDS, such as infections with HIV-1 and related retroviruses of the genus *Lentiviridae,* primate group. Other treatable retroviral infections include, without limitation, human T-cell leukemia (HTLV), tropical spastic paraparesis, and infections caused by avian leukosis virus, bovine leukaemia virus, mouse mammary tumor virus, murine leukaemia virus, human spumavirus, and Mason-Pfizer monkey virus. Other retroviral infections, primarily affecting non-humans that are treatable include, without limitation, non-primate lentiviruses including feline, equine, bovine, and ovine/caprine lentiviruses.

DNA virus infections that may be treated by the uses of the instant invention include hepatitis B, human papilloma, causing warts and cervical lesions, and the herpesviruses which include HHV-1 (HSV1), HHV-2 (HSV2), HHV-3 (VZV), HSV-4 (EBV), HSV-5 (CMV), HSV-7, HSV-7, and HSV-8 (KSV).

Intracellular prokaryotes are susceptible to the mechanism of the gallium compounds of the invention. Macrophage-phagocytosed bacteria which can survive within the macrophage, such as the *Mycobacteria* species *Mycobacterium tuberculosis* and *Mycobacterium leprae,* are also susceptible to the gallium agents of the invention. Obligate intracellular prokaryotes such as *Mycoplasma* species, *Rickettsia* species and *Chlamidia* species are wholly susceptible to the microbe-static, e.g. replication inhibiting, effects of the invention.

It is to be understood that while the invention has been described in conjunction with the preferred specific embodiments thereof, the foregoing description, as well as the examples that follow, are intended to illustrate and not limit the scope of the invention. Other aspects, advantages and modifications will be apparent to those skilled in the art to which the invention pertains.

The following examples are put forth so as to provide those of ordinary skill in the art with a complete disclosure and description of how to make and use the compounds of this invention, and are not intended to limit the scope of what the inventor regards as his invention. Efforts have been made to ensure accuracy with respect to numbers (e.g., amounts, temperature, etc.) but some errors and deviations should be accounted for. Unless otherwise indicated, parts are parts by weight, temperature is in °C and pressure is at or near atmospheric. All solvents were purchased as HPLC or reagent grade and, where appropriate, solvents and reagents were analyzed for purity using common techniques.

In these examples, the following abbreviations have the following meanings:
Å=Angstrom (0.1 nm)
C=Centigrade
kg=kilogram
M=Molar
mg=milligram
ml=milliliter
mm=millimeter
N=Normal
nm=nanometers.
Also, in X-ray fluorescence and diffraction data given in Example 1, the numbers in parentheses after the value reported represent the estimated standard deviation in the last digit.

### Example I

### Preparation of Gallium Ethyl Maltolate

A 1.5M solution of ethyl maltol in chloroform is mixed with an equal volume of a 0.5M solution of gallium nitrate nonohydrate in ethanol to provide a 3:1 molar ratio of ethyl maltol to gallium ions in the mixture. The mixture is stirred for 7 minutes at 22°C. Solid anhydrous sodium carbonate is then added in a 10 molar excess, and stirring continues for an additional ten minutes. When the sodium carbonate is added, a trace of water may sometimes need to be added to facilitate the reaction, which is evidenced by some effervescence. The mixture is then filtered and the filtrate evaporated to give the solid 3:1 complex of ethyl maltol and gallium.

The complex as so produced contains 14.3(1) weight percent gallium by x-ray fluorescence analysis, as predicted for Ga(C₇H₆O₃)₃. The material forms white to pale beige monoclinic crystals with unit cell parameters of about a=7.899(1)Å, b=8.765(1)Å, c=31.626(2)Å, beta=103.253(7) degrees, V=2131 Å³, based on powder x-ray diffraction analysis. The solubility of this compound is measured as about 5 millimolar in distilled deionized water at 23°C. Crystallization from other solvents or under other conditions may produce other crystal structures. Under some conditions, water or other solvents may also be incorporated into the structure.

### Example 2

### Preparation of Gallium Maltol

Maltol is dissolved in chloroform to form a 0.75M solution, and gallium nitrate nonohydrate is dissolved in ethanol to forma 0.5M solution. To 20 ml of the 0.75M maltol solution in chloroform is slowly added, with continuous stirring, 10 ml of the 0.5M gallium nitrate nonohydrate solution in ethanol. The resulting solution is stirred for 5 minutes at 23°C. About 5.5 grams of powdered anhydrous sodium carbonate are added, and stirring continues for additional 12 minutes. The mixture is filtered to remove all solids, and the filtrate is evaporated in a rotary evaporator. The remaining crystalline solid is the 3:1 maltol gallium composition. This composition is analyzed using powder x-ray diffraction and found to consist of orthorhombic crystals with unit cell dimensions of about a=18.52(1)Å, b=16.94(1)Å, c=12.02(1)Å. The solubility of this composition is measured as about 24 millimolar in distilled deionized water at 23°C.

The stability of the neutral 3:1 maltol:gallium complex was studied in aqueous solutions at various pH values. The complex was studied at two concentrations in double distilled deionized water: 2.5 × 10⁻⁶ M and 1.0×10⁻² M. The pH was adjusted by adding either 1N HCl or 1N Na₂CO₃. The stability of the complex was determined using ultraviolet spectroscopy over the region 200-450 nm at 25°C. Several absorption peaks are observed in this range, including those at about 212-217 nm, 248 nm, 273 nm, 318 nm, and 385 nm. An isobesic point occurs over much of the pH range at about 290 nm. In the very dilute solutions (2.5×10⁻⁶ M), the neutral 3:1 complex appears to be stable from about pH 4.5 to 9.5. For the less dilute solutions (1.0×10⁻² M), the determination was more difficult due to the very high absorbance. The stability region appears very similar to that of the highly dilute solution, possibly slightly wider.

### Example 3

### Preparation of Enteric Coated Capsule Formulation

The 3:1 maltol:gallium composition is prepared as described in Example 2. Into a standard size 3 hard gelatin capsule(about 15.5 mm long and 5.8 mm diameter) is added 40 mg of the 3:1 maltol:gallium composition, 10 mg of maltol, and about 190 mg of starch. The capsule is closed and is then coated with a layer of cellulose acetate phthalate/diethyl phthalate using a pilot-scale procedure described by Jones (1970), "Production of enteric coated capsules," *Manufacturing Chemist* & *Aerosol News* 41:43-57, 1970. Acetone is used as a solvent, and a coating thickness of about 35 micrometers is obtained. Such a capsule inhibits the release of its contents (the 3:1 maltol:gallium composition) in the acidic conditions of the stomach, but releases its contents in the small intestine, where the pH is greater than about 5.5.

Other materials well known in the art can be used to enteric coat the capsule by merely substituting for the cellulose acetate phthalate/diethyl phthalate employed in Example 3 above. Such other materials include, by way of example, cellulose acetate phthalate, hydroxypropyl methylcellulose phthalate, poly(vinyl acetate phthalate), hydroxypropyl methylcelluloseacetate succinates, poly(meth)acrylates, and the like.

### Example 4

### Preparation of Capsules Containing a Pharmaceutically Acceptable Buffer

The purpose of this example is to demonstrate the preparation of an orally deliverable pharmaceutical composition containing a neutral complex of gallium and a 3-hydroxy-4-pyrone wherein the means to inhibit dissociation of the complex in the acidic conditions of the stomach is the use of a pharmaceutically acceptable buffer. Specifically, 40 mg of the 3:lmaltol:gallium composition, from about 50 to about 1000 mg (preferably 500 mg) of calcium carbonate, and the balance starch, are added to a standard gelatin capsule. The capsule is then closed to provide a composition of this invention. Such a capsule will inhibit the dissociation of the 3:1 maltol:gallium composition in the acidic conditions of the stomach by raising the pH of the fluid in the stomach.

In view of the above, other neutral complexes of gallium and 3-hydroxy-4-pyrones could be prepared in the methods described above by merely substituting such other 3-hydroxy-4-pyrones for maltol and for ethyl maltol described in the above examples. Similarly, other means to prevent dissociation of the neutral complex could be employed by merely substituting such other means for the means exemplified above.

Specifically, from about 50 to about 1000 mg of other pharmaceutically acceptable buffers can be employed in place of calcium carbonate in the capsules of Example 4. Such other pharmaceutically acceptably buffers include, by way of example, sodium bicarbonate, sodium carbonate and the like.

### Example 5

### Clinical Evaluation of Gallium Maltolate for Treating HIV Infection

Gallium maltolate is evaluated clinically for efficacy in treating HIV infection. The methods of the following reference are used in the evaluation: Kirk et al. (1999), "Combination therapy containing ritonavir plus saquinavir has superior short-term antiretroviral efficacy: a randomized trial," *AIDS* 13(1):9-16. Patients with HIV infection are treated once per day with gelatin capsules containing 250 mg gallium maltolate. Patients are divided randomly and blindly into approximately equal groups that receive either 0 (placebo), 250, 500, or 750 mg/day gallium maltolate for about six months, together with 300 mg AZT twice per day, 200 mg ddI twice per day, and 800 mg indinavir every eight hours. Patients are medically monitored for symptoms of HIV infection throughout the study. In addition, blood serum samples are obtained from the patients at days 0, 30, 90, and 180 and are assayed for CD4 T-cell count by routine methods and for HIV-1 RNA (viral load assay) using the Roche AMPLICOR ultra-assay (Sun et al. (1998), "Ultrasensitive reverse transcription-PCR assay for quantitation of human immunodeficiency virus type 1 RNA in plasma," *J Clin. Microbiol.* 36(10):2964-2969). Experimental work conducted according to the documented procedures shows that gallium maltolate and related compounds of this invention are effective for treating HIV infection.

### Example 6

### Clinical Evaluation of Gallium Maltolate for Treating Hepatitis B Infection

Gallium maltolate is evaluated clinically for efficacy in treating Hepatitis B infection. The methods employed in the clinical studies discussed in the following references are used in the evaluation: Yao et al. (1999), "Treatment of Chronic Hepatitis B: New Antiviral Therapies," *Curr. Gastroenterol. Rep.* 1(1):20-26; Hoofnagle et al. (1997), "New therapies for chronic hepatitis B," *J. Viral Hepat.* 4 Suppl., 1:41-50. Patients with hepatitis B infection are treated once per day with gelatin capsules containing gallium maltolate. Patients are divided randomly and blindly into two approximately equal groups of four approximately equal subgroups that receive either 0 (placebo), 250, 500, or 750 mg/day gallium maltolate for about six months. The first four groups receive the gallium maltolate dose together with 5 million IU interferon by injection once per day. The second four groups receive the gallium maltolate dose together with 5 million IU interferon by injection once per day, and 150 mg lamuvidine twice per day. Patients are also medically monitored for liver disease, including liver function testing and examination for medical symptoms of hepatitis B infection throughout the study, with careful attention to the possibility that the drugs themselves may cause some liver disease and elevate the liver function tests. Thus liver function testing is done after the first week, the second week and every two weeks thereafter. In addition, liver histopathology is studied using biopsy and immunostaining and *in situ* hybridization for pertinent antigens and nucleic acids, with liver biopsy samples obtained from the patients at days 0, 30, 90, and 180 days. Blood is assayed after the first week, the second week and every two weeks for pertinent antigens, including the hepatitis B surface antigen (HbsAg) and "e" antigen (HbeAg) by routine methods. Successful treatment may convert patients with chronic hepatitis B from a replicating (HBeAg positive) to non-replicating (HBeAg negative) state, and some patients may even be cured as assessed by the loss of HbsAg altogether.

### Example 7

### Clinical Evaluation of Gallium Maltolate for Mycoplasmal Pneumonia Infection in HIV Infected Patients

Pneumonia caused by *Mycoplasma pneumonia* is termed "walking pneumonia" and often afflicts HIV infected patients, but also affects immunocompetent individuals. It is often treated with bacteriocidal agents, but tetracycline is a bacteriostatic agent used in treating it. The action of gallium is appears somewhat closer to static rather than cidal. Cidal and static agents are not combined in antimicrobial therapy for prokaryotes, thus gallium could be used with tetracyclines for refractory mycoplasmal pneumonias, but these are only likely to be seen with HIV patients. As tetracycline inhibits protein synthesis at the prokaryotic ribosome, synergy with the drastically different mechanistic action of gallium is expected. As th course of HIV disease is much longer than the normal successful course of treatment for the walking pneumonia, the gallium treatment will have little effect on the HIV or in assisting in reconstituting the immune system as by enhancing highly aggressive antretroviral therapy (HAART).

HIV patients on a HAART (excluding the gallium) regime identical to that described in Example 5 that have mycoplasmal pneumonia are randomly and blindly divided into two approximately equal groups. The two groups are then divided in the same random manner into four subgroups each, for different gallium doses (including the placebo dose). Each group is then treated identically with gallium maltolate as described in Example 5. The first group receives tetracycline 500 mg four times daily to treat the pneumonia in addition to the gallium while the second group only receives the gallium in addition to the standard HAART. Experimental work conducted according to the documented procedures shows that gallium maltolate and related compounds of this invention are effective for treating mycoplasmal pneumonia in HIV patients. As expected the combination of gallium with tetracyclines is more effective than either agent alone, and synergy is also demonstrated by several computational data analysis techniques including determination by MacSynergy II® computer program.

### Example 8

### Clinical Evaluation of Gallium Maltolate for Treating Herpes Simplex (I or 11) Infection and Recurrences

Gallium maltolate is evaluated clinically for efficacy in treating Herpes Simplex infection in HIV negative individuals who show no signs of immune dysfunction of any type. Patients with Herpes Simplex infection are treated once per day with gelatin capsules containing gallium maltolate. The population of patients is divided into subpopulation with recurrent Herpes Simplex and acute Herpes Simplex infections respectively. These different subpopulations are divided randomly and blindly into two approximately equal groups with four approximately equal subgroups that receive either 0 (placebo), 250, 500, or 750 mg/day gallium maltolate for about six months. The first group of each subpopulation receive the gallium maltolate dose together with 200 mg acyclovir five times per day. The second group of each subpopulation receive the gallium maltolate dose together with 500 mg ganciclovir twice per day. Patients are medically monitored for resolution recurrences of latent Herpes Simplex infection, or medical symptoms of acute Herpes Simplex infection throughout the study, with the monitoring including *in situ* hybridization of skin lesions for HSV DNA. In addition for the acute Herpes Simplex infected group, blood serum samples are obtained from the patients at days 0, 3, 7, and 10 and are assayed for HSV particle by routine methods and for Herpes Simplex DNA (viral load assay) using PCR. Experimental work conducted according to the procedures shows that gallium maltolate and related compounds of this invention are effective for treating Herpes Simplex infection, including recurrent sores as well as more serious systemic disease. As expected the combination of gallium with acyclovir or ganciclovir is more effective than either agent alone, and synergy of gallium with both nicleoside analogs is also demonstrated by several computational data analysis techniques including determination by MacSynergy II® computer program.

### Example 9

### Clinical Evaluation of Gallium Maltolate for Treating Herpes Varicella-Zoster (VZV) Infection and Recurrences

Gallium maltolate is evaluated clinically for efficacy in treating Varicella-Zoster (VZV) infection in HIV negative individuals who show no signs of immune dysfunction of any type. Patients with VZV infection are treated once per day with gelatin capsules containing gallium maltolate. The population of patients is divided into subpopulation with recurrent VZV (shingles) and acute VZV infections (chickenpox) respectively. These different subpopulations are divided randomly and blindly into two approximately equal groups with four approximately equal subgroups that receive either 0 (placebo), 250, 500, or 750 mg/day gallium maltolate for about six months. The first group of each subpopulation receive the gallium maltolate dose together with 800 mg acyclovir five times per day. The second group of each subpopulation receive the gallium maltolate dose together with 500 mg ganciclovir twice per day. Patients are medically monitored for resolution reoccurrences of latent VZV infection, or medical symptoms of acute VZV infection throughout the study, with the monitoring including *in situ* hybridization of skin lesions for VZV DNA. In addition for the acute VZV infected group, blood serum samples are obtained from the patients at days 1-5 and are assayed for VZV particles by routine methods and for VZV DNA (viral load assay) using PCR. Experimental work conducted according to the procedures shows that gallium maltolate and related compounds of this invention are effective for treating VZV infection, including recurrent sores as well as more serious systemic disease. As expected the combination of gallium with acyclovir or ganciclovir is more effective than either agent alone, and synergy of gallium with both nicleoside analogs is also demonstrated by several computational data analysis techniques including determination by MacSynergy II® computer program.

### Example 10

### Clinical Evaluation of Gallium Maltolate for Treating Epstein-Barr Virus (EBV) Infection

Gallium maltolate is evaluated clinically for efficacy in treating EBV infection in HIV negative individuals who show no signs of immune dysfunction of any type, except for the EBV mononucleosis. Some of the methods employed in the clinical studies discussed in the following references are used in the evaluation: Schneider et al. (2000), "Regression of Epstein-Barr virus-associated lymphoproliferative disorders in patients with acquired immunodeficiency syndrome during therapy with foscamet," *Ann. Hematol.* 79(4):214-6. Patients with EBV infection are treated once per day with gelatin capsules containing gallium maltolate. The patients are divided randomly and blindly into two approximately equal groups with four approximately equal subgroups that receive either 0 (placebo), 250, 500, or 750 mg/day gallium maltolate for about six months. The first group receive the gallium maltolate dose together with 500 mg ganciclovir twice per day. The second group receive the gallium maltolate dose together with 5500 mg foscarnet, IV infusion over 1.5-2 hr., twice per day. Patients are medically monitored for resolution of EBV infection, with histopathologic examination of hematologic smears to assess characteristic cells an lymphocyte levels. In addition blood serum samples are obtained from the patients at days 1, 3, 7, 10, 14, 17, 21, 24 and 28 and are assayed for EBV particles by routine methods and for EBV DNA (viral load assay) using PCR. Experimental work conducted according to the procedures shows that gallium maltolate and related compounds of this invention are effective for treating EBV infection, including recurrent sores as well as more serious systemic disease. As expected the combination of gallium with acyclovir or ganciclovir is more effective than either agent alone, and synergy of gallium with both nicleoside analogs is also demonstrated by several computational data analysis techniques including determination by MacSynergy II® computer program.

### Example 11

### Clinical Evaluation of Gallium Maltolate for Treating Co-infection by HIV and Hepatitis B

The orally administrable, highly Tf-bound, well distributed gallium compounds of the instant invention are expected to be synergistic with existing HAART regimes, potentiating more successful reconstitution of the immune system in terms of regaining for a higher proportion of infected patients, and maintaining for a longer time specific immune responses against HIV and other intracellular microbes. By doing so the compounds of the instant invention also potentiate or enhance the potentiality for use of biological humoro-hormonal agents to stimulate the specific immune responses against HIV or another intracellular pathogen.

The recent addition of combination therapies adding a nucleoside analog to interferon-alpha (α-interferon) in treatment of hepatitis B and hepatitis C, suggest another significant advantage of the gallium compounds of the instant invention in the combination treatment of simultaneous infection by HIV and another virus. Because the compounds of the instant invention are effective against any intracellular pathogen which makes DNA during its life cycle by disrupting the iron metabolism of the host cell, they are effective against a combination of viruses.

Hepatitis B and hepatitis C treatments are commonly known to have traditionally been based on interferon alone with nucleoside analogs being added as combination antiviral therapy agents only recently; persons with HIV disease are often infected with one and sometimes with both, and once their immune system is compromised the administration of interferon becomes futile if not counterproductive. Thus the instant invention offers a synergistic combination with nucleoside analogs which may, without interferon, be capable of controlling hepatitis B in HIV patients and combines with the anti-HIV therapy synergystically to improve specific immunity against the hepatitis virus, and thereby potentiate the later addition of interferon-alpha to the anti-hepatitis regimen if sufficient improvement of immune function as measured by CD4+ levels or otherwise is observed.

Gallium maltolate is evaluated clinically for efficacy in treating hepatitis B co-infection of a HIV-1 patients. In addition to the methods used in Example 5 for evaluating the HIV disease, the methods employed in the clinical studies discussed in the following references are used in the evaluation: Yao et al. (1997), "Treatment of Chronic Hepatitis B: New Antiviral Therapies," *Curr. Gastroenterol. Rep.* 1(1):20-26; Hoofnagle et al. (1997), "New therapies for chronic hepatitis B," *supra.*

Patients are treated once per day with gelatin capsules containing gallium maltolate. The population of patients with hepatitis B and HIV infection are first divided into two subpopulations groups based on CD4+ counts (above 350/mm³ and below 350/mm³). The subpopulations are randomly and blindly divided into two groups each randomly subdivided into of four subgroups for a total of sixteen approximately equal subgroups that receive either 0 (placebo), 250, 500, or 750 mg/day gallium maltolate for about six months. All subgroups receive the identical regimen of non-gallium antiretroviral therapy as described in Example 5 (a HAART regime). As combination of interferon therapy with nucleoside analogs for hepatitis is relatively recent interferon plus gallium alone is compared to interferon plus gallium plus nucleoside analog. Specifically interferon-alpha (α-interferon) is used. The most commonly used nucleoside analog for hepatitis B is lamivudine, and newer alternatives include famciclovir, lobucavir and adefovir dipivoxil.

In each subpopulation, the first group's four subgroups from each subpopulation (above 350/mm³ and below 350/mm³) additionally receive the gallium maltolate dose together with 10 million IU interferon by injection once per day. This is twice the dose used in non-HIV infected individuals in Example 6, because of the HIV disease effects on immunity. In each subpopulation, the second group's four groups receive the gallium maltolate dose together with 150 mg lamuvidine twice per day. Patients are monitored for HIV disease precisely as described in Example 5. Patients are also medically monitored for liver disease, including liver function testing and examination for medical symptoms of hepatitis B infection throughout the study, with careful attention to the possibility that the drugs themselves may cause some liver disease and elevate the liver function tests. Thus liver function testing is done after the first week, the second week and every two weeks thereafter. In addition, liver histopathology is studied using biopsy and immunostaining and *in situ* hybridization for pertinent antigens and nucleic acids, with liver biopsy samples obtained from the patients at days 0, 30, 90, and 180 days. Blood is assayed after the first week, the second week and every two weeks for pertinent antigens, including the hepatitis B surface antigen (HbsAg) and "e" antigen (HbeAg) by routine methods. Successful treatment may convert patients with chronic hepatitis B from a replicating (HBeAg positive) to non-replicating (HBeAg negative) state, and some patients may even be cured as assessed by the loss of HbsAg altogether, but the likelihood of a cure with HIV disease is appreciated to be diminished. Lamuvidine has been shown to be effective against HIV, but the newer nucleoside analogs being used for hepatitis B are also likely to be effective against HIV. The appropriate dosing for the nucleoside analogs which can be substituted for lamuvidine follow: famciclovir: 500 mg twice daily; lobucavir: 400 mg once daily; adefovir dipivoxil (adefovir): 120 mg once daily. The adefovir dose is four times the dosage demonstrated in trials as effective against hepatitis B alone and the higher of the two dosages (60 mg and 120 mg per day) being evaluated for HIV disease alone

Experimental work conducted according to the documented procedures shows that gallium maltolate and related compounds of this invention are effective in combination with interferon and interferon plus nucleoside analogs, and synergistic with both, in treating hepatitis B infection. The nucleoside analogs used in the treatment of hepatitis are also synergistic with the HAART regime in their effects upon the HIV infection, as is the gallium, as determined by MacSynergy II® computer program. The low CD4+ group is less responsive overall, as expected, but for those in the low CD4+ subpopulation who experience a CD4+ level surge, the rise in CD4+ cells correlates with increased rate of amelioration of the hepatitis, a phenomenon observed to a lesser extent in the high CD4+ group (less proportionate CD4+ increase seen). Those in the low CD4+ group who do not experience a significant rise in their CD4+ levels do not experience an acceleration of the resolution of the hepatitis, an although responding to the treatment are the least responsive.

### Example 12

### Clinical Evaluation of Gallium Maltolate for Prevention of CM V Disease in HI V Patients

CMV retinitis is a common complication of HIV disease, but CMV has also been shown to cause other diseases in immunocompromised individuals, including interstitial pneumonia and acute virulent CMV hepatitis. Nucleoside analogs effective against HIV are known to be effective against other retroviruses, but different nucleoside analogs are typically used and more effective against members of the human herpesvirus family which includes eight members previously described, and nucleoside analogs differing from the antiretroviral nucleoside analogs are commonly used to treat the herpesviruses.

Because of the mechanism of action of the gallium compounds of the instant invention, specifically cross organismal efficacy, and synergy with other respective antiviral regimes, treatment of multiple infections is enhanced in a broad manner. For example, an individual with HIV disease and CMV on existing HAART may not respond to interferon, because of immune system compromise. Existing HAART regime plus ganciclovir, even if tolerated with the HAART despite the cumulative side effects on the host's cells of the ganciclovir and antiretroviral nucleoside analogs, is not a combination therapy for the CMV disease. Adding the gallium compounds of the instant invention to the HAART plus ganciclovir regime, will synergistically enhance the HAART, and also synergistically complement the acyclovir treatment for the CMV. Furthermore the enhanced HAART (EHAART) may be able to reconstitute some specific immune function against both HIV and CMV, potentiating addition of interferon or leukotrienes to the regime, and by helping reconstitute the specific immune response, making a further synergistic contribution to the treatment of the CMV disease in the presence of HIV disease.

In this example, prevention of CMV disease rather than its treatment will be studied. Only chemo-prevention by antivirals, rather than with immune stimulating agents such as interferons, because interferon requires regular injection. Foscarnet is an antiviral only for injection, but it is studied for comparison with oral antivirals.

Gallium maltolate is evaluated clinically for efficacy in preventing CMV disease in HIV patients. In addition to the methods used in Example 5 for evaluating the HIV disease, some of the methods employed in the following reference are used in the evaluation: Monkemuller et al. (2000), "Esophageal ulcer caused by cytomegalovirus: resolution during combination antiretroviral therapy for acquired immunodeficiency syndrome," *South Med. J.* 93(8):818-20.

Patients are treated once per day with gelatin capsules containing gallium maltolate. The population of patients with CMV (almost universal, seropositive or culture positive patients) and HIV infection are first divided into two subpopulations groups based on CD4+ counts (above 350/mm³ and below 350/mm³). The subpopulations are randomly and blindly divided into two groups each randomly subdivided into of four subgroups for a total of sixteen approximately equal subgroups that receive either 0 (placebo), 250, 500, or 750 mg/day gallium maltolate for about six months. All subgroups receive the identical regimen of non-gallium antiretroviral therapy as described in Example 5 (a HAART regime). The commonly used nucleoside analog for CMV is ganciclovir, with foscavir used in cases where ganciclovir is ineffective, and the combination used to treat refractory CMV retinitis and other conditions. Foscamet must be infused slowly to avoid adverse reactions. In each subpopulation, the first group's four subgroups from each subpopulation (above 350/mm³ and below 350/mm³) additionally receive the gallium maltolate dose together with 5500 mg foscarnet, IV infusion over 1.5-2 hr., twice per day. In each subpopulation, the second group's four subgroups receive the gallium maltolate dose together with 1000 mg ganciclovir four times per day. Patients are monitored for HIV disease precisely as described in Example 5. Patients are also medically monitored for CMV disease, including testing and examination for medical symptoms of CMV disease, such as CMV retinitis, throughout the study. Any mucosal or skin lesions are studied by *in situ* hybridization for CMV DNA. Regular comprehensive medical examinations taking blood and urine samples and lung bronchoalveolar lavage samples for histopathologic, immunologic and virologic evaluation by routine methods 0, 30, 60, 90, 120, 150, 180 and every 30 days thereafter so that the patients are followed over at least the course of one year. Blood and urine are assayed after the first week, the second week and every two weeks thereafter for pertinent antigens and CMV DNA by routine methods, including PCR for CMV DNA.

Experimental work conducted according to the documented procedures shows that gallium maltolate and related compounds of this invention are effective in combination with either foscarnet or ganciclovir, and synergistic with both, in preventing CMV disease. The nucleoside analogs used in the treatment of CMV are also synergistic with the HAART regime in their effects upon the HIV infection, as is the gallium, as determined by MacSynergy II® computer program. The low CD4+ subpopulation is less responsive overall, as expected, but for those in the low CD4+ group who experience a CD4+ level increase over the treatment, the rise in CD4+ cells correlates with lowered rates of CMV disease, a phenomenon observed to a lesser extent in the high CD4+ group. Those in the low CD4+ group who do not experience a significant rise in their CD4+ levels are the most likely to develop CMV disease. Prevention of CMV disease with only oral agents is possible with gallium plus ganciclovir being adequate and effecting better results than placebo plus ganciclovir.

### Example 13

### Clinical Evaluation of Gallium Maltolate for Prevention of EBV or KSV Neoplasms in HIV Patients

The observed resolution, with reconstitution by combination antiretroviral therapy of the immune response in a HIV patient, of CMV ulceration of the esophagus without any anti-CMV therapy illustrates the importance of immune response for overcoming pathologies caused by herpesvirus family members (Monkemuller et al. (2000), *supra*). Addition of the gallium compounds of the instant invention in the treatment of patients having AIDS and an opportunistic viral infection would permit a much higher resolution rate because of both enhancement of the combination antiretroviral therapy leading to a better reconstitution of the immune system for a greater proportion of patients, and direct activity against the CMV lesion. As CMV retinitis is a leading cause of blindness in AIDS patients, the addition of the gallium compounds of the instant invention to the orally administered anti-retroviral and the anti-CMV pharmacotherapy, possibly with topical application of gallium maltolate to the eyes or intraocular injection of gallium maltolate preparations, promises to help more AIDS patients retain their sight for longer.

Both EBV and KSV cause neoplasms, with both causing lymphomas and KSV (HHV-7) along with HHV-7, possibly in concert with HHV-6 associated with the pathogenesis of Kaposi Sarcoma. Most humans carry all these viruses. A HIV population could be studied without determining whether any of the patients are seropositive or culturable for these pathogens, or preferably a group of HIV patients who are seropositive or culture positive for both EBV and KSV can be identified. Applying the methods of Example 12 with additional guidance from Schneider et al. (2000), *supra,* yields a protocol for evaluating the contribution of the gallium compounds of the invention in prevention of opportunistic neoplasms caused by the herpesviruses with combination antiviral therapy.

Using essentially the same protocol as Example 12, but with an HIV infected patient population that is seropositive or culture positive for both EBV and KSV and substituting the specific assays such as PCR, serodetection for the EBV and KSV viruses, the role of gallium as a combination agent in preventing the neoplasms caused by members of the herpesvirus family in HIV disease can be evaluated. The subpopulations are divided according to CD4 counts as in Example 12. Each subpopulation's first group is treated in addition to the foscarnet with interferon-alpha. Each subpopulation's second group is treated in addition to the ganciclovir with the same doses of foscarnet and interferon-alpha as the first group. The dosages of the foscarnet and are as in Example 12, as are the dosages for the gallium maltolate for each groups four subgroups. Thus within each subpopulation, gallium plus foscarnet plus traditional HAART is compared to gallium plus foscarnet plus traditional HAART plus ganciclovir. An all oral regimen would be preferable but success would be unlikely. The dosage level for the interferon-alpha used is that used for treating Kaposi Sarcoma (20 million IU every day by injection), a dose that may require adjustment for tolerance in some patients. As in Example 12 the study should last at least a year, and as long as the patient population can be followed while still numbering enough individuals to yield statistically significant results.

Experimental work conducted according to the described procedures shows that gallium maltolate and related compounds of this invention are effective in combination with foscamet, interferon and ganciclovir, and synergistic with all, in preventing herpesvirus neoplasia in HIV infected patients. The nucleoside analogs and interferon-alpha in the preventive treatment are also all synergistic with the HAART regime in their effects upon the HIV infection, as is the gallium, as determined by MacSynergy II® computer program. The low CD4+ subpopulation is less responsive overall, as expected, but for those in the low CD4+ group who experience a CD4+ level increase over the treatment, the rise in CD4+ cells correlates with lowered rates of neoplasm, a phenomenon observed to a lesser extent in the high CD4+ group. Those in the low CD4+ group who do not experience a significant rise in their CD4+ levels are the most likely to develop neoplasms. Prevention of opportunistic herpesvirus associated neoplasms is possible with gallium combined with foscarnet, ganciclovir and interferon in the high CD4+ group producing the best results. Early antiviral treatment is thus desirable to keep CD4+ levels high enough to permit such preventative strategies later, arguing for the earliest possible institution of oral antiviral combination therapy after diagnosis of HIV infection.

## Claims

1. Use of gallium in the form of a neutral 3:1 (hydroxypyrone :gallium) complex in which the hydroxypyrone is either unsubstituted or substituted with one through three C₁-C₆ alkyl substituents which may be the same or different for the manufacture of a medicament for treating or preventing an obligate intracellular prokaryote, DNA virus, or retrovirus infection in an individual, wherein said medicament is adapted to administer a therapeutically effective amount of gallium to the individual, and wherein the therapeutically effective amount is such that a blood plasma gallium concentration is provided that is sufficient to enable treatment or prevention of the infection.

2. The use of claim 1, wherein the complex is administered orally.

3. The use of claim 2, wherein the complex is administered in a dose of approximately 10 to 2500 mg per day.

4. The use of claim 1, wherein the hydroxypyrone is selected from the group consisting of 3-hydroxy-4-pyrone, 3-hydroxy-2-methyl-4-pyrone, 3-hydroxy-2-ethyl-4-pyrone, and 3-hydroxy-6-methyl-4-pyrone.

5. The use of claim 4, wherein the hydroxypyrone is selected from the group consisting of 3-hydroxy-2-methyl-4-pyrone and 3-hydroxy-2-ethyl-4-pyrone.

6. The use of claim 5, wherein the hydroxypyrone is 3-hydroxy-2-methyl-4-pyrone.

7. The use of claim 1, wherein the medicament further includes an additional active agent.

8. The use of claim 7, where the infection is by a virus and the additional active agent is a nucleoside analog active against the infecting virus.

9. The use of claim 7, where the infection is by an obligate intracellular prokaryote and the additional active agent is a bacteriostatic antibiotic.

10. The use of claim 8, where the infection is by a retrovirus and the composition further includes a protease inhibitor.

11. The use of claim 1, wherein the infection is by a prokaryote or viral infection associated with AIDS in an individual also infected with HIV.

12. The use of claim 1, wherein the infection is retroviral and an HIV infection.

13. The use of claim 12, wherein the medicament further includes one or more additional active agents selected from the group consisting of nucleoside analogs, protease inhibitors and non-nucleoside reverse transcriptase inhibitors.

14. The use of claim 13, wherein the additional active agents include one or more agents selected from the group consisting of nucleoside analogs and non-nucleoside reverse transcriptase inhibitors.

15. The use of claim 14, where the one or more nucleoside analogs are selected from the group consisting of AZT, ddI, and ddC.

16. The use of claim 14, where the medicament additionally includes a protease inhibitor.

17. The use of claim 16, where a biomolecule that stimulates the immune system is additionally administered.

18. The use of claim 1, wherein the infection is a human herpesvirus infection.

19. The use of claim 18, wherein the medicament further includes one or more nucleoside analogs effective against members of the herpesvirus family.

20. The use of claim 19, where the one or more nucleoside analogs effective agains members of the herpesvirus family are selected from the group consisting of acyclovir, gancyclovir, and foscarnet.

21. The use of claim 20, where a biomolecule that stimulates the immune system is additionally administered.

22. The use of claim 1, wherein the infection is a hepatitis B infection.

23. The use of claim 1, wherein the medicament is administered topically and the carrier is suited to topical drug administration.

24. The use of claim 1, wherein the medicament is administered to the eye and the carrier is suited to topical drug administration to the eye.

25. Use of gallium in the form of a neutral 3:1 (hydroxypyrone:gallium) complex in which the hydroxypyrone is either unsubstituted or substituted with one through three C₁-C₆ alkyl substituents which may be the same or different for the manufacture of a medicament for treating or preventing a co-infection by an obligate intracellular prokaryote, DNA virus, or retrovirus in a mammalian individual, infected with HIV, wherein said medicament is adapted to administer a therapeutically effective amount of gallium to the mammalian individual, and wherein the therapeutically effective amount is such that a blood plasma gallium concentration is provided that is sufficient to enable treatment or prevention of the infection and co-infection.

## Patentansprüche

1. Verwendung von Gallium in der Form eines neutralen 3:1 (Hydroxypyron:Gallium)-Komplexes, bei dem der Hydroxypyron entweder unsubstituiert oder mit einem bis drei C₁-C₆-Alkylsubstituenten, die gleich oder unterschiedlich sein können, substituiert ist, zur Herstellung eines Medikaments zur Behandlung oder Prävention einer Infektion durch einen obligatorisch intrazellulären Prokaryonten, einen DNA-Virus oder einen Retrovirus in einem Individuum, wobei das Medikament so hergerichtet wird, dass eine therapeutisch wirksame Menge Gallium an das Individuum verabreicht wird, und wobei die therapeutisch wirksame Menge so ist, dass eine Blutplasmagalliumkonzentration bereitgestellt wird, die ausreichend ist, um eine Behandlung oder Prävention der Infektion zu ermöglichen.

2. Verwendung nach Anspruch 1, wobei der Komplex oral verabreicht wird.

3. Verwendung nach Anspruch 2, wobei der Komplex in einer Dosis von etwa 10 bis 2500 mg pro Tag verabreicht wird.

4. Verwendung nach Anspruch 1, wobei der Hydroxypyron aus der Gruppe bestehend aus 3-Hydroxy-4-pyron, 3-Hydroxy-2-methyl-4-pyron, 3-Hydroxy-2-ethyl-4-pyron und 3-Hydroxy-6-methyl-4-pyron ausgewählt ist.

5. Verwendung nach Anspruch 4, wobei der Hydroxypyron aus der Gruppe bestehend aus 3-Hydroxy-2-methyl-4-pyron und 3-Hydroxy-2-ethyl-4-pyron ausgewählt ist.

6. Verwendung nach Anspruch 5, wobei der Hydroxypyron 3-Hydroxy-2-methyl-4-pyron ist.

7. Verwendung nach Anspruch 1, wobei das Medikament ferner einen zusätzlichen Wirkstoff umfasst.

8. Verwendung nach Anspruch 7, wobei die Infektion durch einen Virus verursacht wird und der zusätzliche Wirkstoff ein gegen den infizierenden Virus wirksames Nukleosidanalogon ist.

9. Verwendung nach Anspruch 7, wobei die Infektion durch einen obligatorisch intrazellulären Prokaryonten verursacht wird und der zusätzliche Wirkstoff ein bakteriostatisches Antibiotikum ist.

10. Verwendung nach Anspruch 8, wobei die Infektion durch einen Retrovirus verursacht wird und die Zusammensetzung ferner einen Proteaseinhibitor umfasst.

11. Verwendung nach Anspruch 1, wobei die Infektion durch einen Prokaryonten verursacht wird oder eine mit AIDS verbundene virale Infektion in einem Individuum, das auch mit HIV infiziert ist, ist.

12. Verwendung nach Anspruch 1, wobei die Infektion retroviral ist und eine HIV-Infektion ist.

13. Verwendung nach Anspruch 12, wobei das Medikament ferner einen oder mehrere zusätzliche Wirkstoffe, ausgewählt aus der Gruppe bestehend aus Nukleosidanaloga, Proteaseinhibitoren und Nicht-Nukleosid-Reverse-Transkriptase-Inhibitoren, umfasst.

14. Verwendung nach Anspruch 13, wobei die zusätzlichen Wirkstoffe ein oder mehrere Mittel, ausgewählt aus der Gruppe bestehend aus Nukleosidanaloga und Nicht-Nukleosid-Reverse-Transkriptase-Inhibitoren, umfassen.

15. Verwendung nach Anspruch 14, wobei der eine oder die mehreren Nukleosidanaloga ausgewählt sind aus der Gruppe bestehend aus AZT, ddl und ddC.

16. Verwendung nach Anspruch 14, wobei das Medikament zusätzlich einen Proteaseinhibitor umfasst.

17. Verwendung nach Anspruch 16, wobei zusätzlich ein das Immunsystem stimulierendes Biomolekül verabreicht wird.

18. Verwendung nach Anspruch 1, wobei die Infektion eine humane Herpesvirus-Infektion ist.

19. Verwendung nach Anspruch 18, wobei das Medikament ferner ein oder mehrere gegen Mitglieder der Herpesvirus-Familie wirksame Nukleosidanaloga umfasst.

20. Verwendung nach Anspruch 19, wobei das eine oder die mehreren gegen Mitglieder der Herpesvirus-Familie wirksamen Nukleosidanaloga ausgewählt sind aus der Gruppe bestehend aus Acyclovir, Gancyclovir und Foscarnet.

21. Verwendung nach Anspruch 20, wobei zusätzlich ein das Immunsystem stimulierendes Biomolekül verabreicht wird.

22. Verwendung nach Anspruch 1, wobei die Infektion eine Hepatitis B-Infektion ist.

23. Verwendung nach Anspruch 1, wobei das Medikament topisch verabreicht wird und die Trägersubstanz für eine topische Arzneimittelverabreichung geeignet ist.

24. Verwendung nach Anspruch 1, wobei das Medikament an das Auge verabreicht wird und die Trägersubstanz für eine topische Arzneimittelverabreichung an das Auge geeignet ist.

25. Verwendung von Gallium in der Form eines neutralen 3:1 (Hydroxypyron:Gallium)-Komplexes, bei dem der Hydroxypyron entweder unsubstituiert oder mit einem bis drei C₁-C₆-Alkylsubstituenten, die gleich oder unterschiedlich sein können, substituiert ist, zur Herstellung eines Medikaments zur Behandlung oder Prävention einer Co-Infektion durch einen obligatorisch intrazellulären Prokaryonten, einen DNA-Virus oder einen Retrovirus in einem Säugetierindividuum, das mit HIV infiziert ist, wobei das Medikament so hergerichtet wird, dass eine therapeutisch wirksame Menge von Gallium an das Säugetierindividuum verabreicht wird, und wobei die therapeutisch wirksame Menge so ist, dass eine Blutplasmagalliumkonzentration bereitgestellt wird, die ausreichend ist, um eine Behandlung oder Prävention der Infektion und Co-Infektion zu ermöglichen.

## Revendications

1. Utilisation de gallium sous la forme d'un complexe (hydroxypyrone:gallium) 3:1 neutre, dans lequel l'hydroxypyrone est soit non substituée, soit substituée par un à trois substituants alkyles en C₁ à C₆ qui peuvent être identiques ou différents, pour la fabrication d'un médicament pour le traitement ou la prévention d'une infection par un procaryote intracellulaire obligé, un virus à ADN ou un rétrovirus chez un individu, dans laquelle ledit médicament est adapté pour administrer une quantité thérapeutiquement efficace de gallium à l'individu, et dans laquelle la quantité thérapeutiquement efficace est telle qu'une concentration en gallium dans le plasma sanguin est fournie qui est suffisante pour permettre le traitement ou la prévention de l'infection.

2. Utilisation selon la revendication 1, dans laquelle le complexe est administré par voie orale.

3. Utilisation selon la revendication 2, dans laquelle le complexe est administré en une dose d'environ 10 à 2500 mg par jour.

4. Utilisation selon la revendication 1, dans laquelle l'hydroxypy-rone est choisie dans le groupe constitué par la 3-hydroxy-4-pyrone, la 3-hydroxy-2-méthyl-4-pyrone, la 3-hydroxy-2-éthyl-4-pyrone et la 3-hydroxy-6-méthyl-4-pyrone.

5. Utilisation selon la revendication 4, dans laquelle l'hydroxypyrone est choisie dans le groupe constitué par la 3-hydroxy-2-méthyl-4-pyrone et la 3-hydroxy-2-éthyl-4-pyrone.

6. Utilisation selon la revendication 5, dans laquelle l'hydroxypyrone est la 3-hydroxy-2-méthyl-4-pyrone.

7. Utilisation selon la revendication 1, dans laquelle le médicament comprend en outre un agent actif supplémentaire.

8. Utilisation selon la revendication 7, dans laquelle l'infection est provoquée par un virus et l'agent actif supplémentaire est un analogue de nucléoside actif contre le virus infectieux.

9. Utilisation selon la revendication 7, dans laquelle l'infection est provoquée par un procaryote intracellulaire obligé et l'agent actif supplémentaire est un antibiotique bactériostatique.

10. Utilisation selon la revendication 8, dans laquelle l'infection est provoquée par un rétrovirus et la composition comprend en outre un inhibiteur de protéases.

11. Utilisation selon la revendication 1, dans laquelle l'infection est provoquée par un procaryote ou est une infection virale associée au SIDA chez un individu également infecté par le VIH.

12. Utilisation selon la revendication 1, dans laquelle l'infection est une infection rétrovirale et une infection par le VIH.

13. Utilisation selon la revendication 12, dans laquelle le médicament comprend en outre un ou plusieurs agents actifs supplémentaires choisis dans le groupe constitué par les analogues de nucléosides, les inhibiteurs de protéases et les inhibiteurs de la transcriptase inverse non nucléosidiques.

14. Utilisation selon la revendication 13, dans laquelle les agents actifs supplémentaires comprennent un ou plusieurs agents choisis dans le groupe constitué par les analogues de nucléosides et les inhibiteurs de la transcriptase inverse non nucléosidiques.

15. Utilisation selon la revendication 14, dans laquelle lesdits un ou plusieurs analogues de nucléosides sont choisis dans le groupe constitué par l'AZT, le ddI et le ddC.

16. Utilisation selon la revendication 14, dans laquelle le médicament comprend en outre un inhibiteur de protéases.

17. Utilisation selon la revendication 16, dans laquelle une biomolécule qui stimule le système immunitaire est administrée en plus.

18. Utilisation selon la revendication 1, dans laquelle l'infection est une infection par un virus de l'herpès humain.

19. Utilisation selon la revendication 18, dans laquelle le médicament comprend en outre un ou plusieurs analogues de nucléosides efficaces contre des membres de la famille des virus de l'herpès.

20. Utilisation selon la revendication 19, dans laquelle lesdits un ou plusieurs analogues de nucléosides efficaces contre des membres de la famille des virus de l'herpès sont choisis dans le groupe constitué par l'acyclovir, le gancyclovir et le foscarnet.

21. Utilisation selon la revendication 20, dans laquelle une biomolécule qui stimule le système immunitaire est administrée en plus.

22. Utilisation selon la revendication 1, dans laquelle l'infection est une infection par l'hépatite B.

23. Utilisation selon la revendication 1, dans laquelle le médicament est administré localement et le véhicule est approprié pour une administration de médicament locale.

24. Utilisation selon la revendication 1, dans laquelle le médicament est administré à l'oeil et le véhicule est approprié pour une administration de médicament locale à l'oeil.

25. Utilisation de gallium sous la forme d'un complexe (hydroxypyrone:gallium) 3:1 neutre, dans lequel l'hydroxypyrone est soit non substituée, soit substituée par un à trois substituants alkyles en C₁ à C₆ qui peuvent être identiques ou différents, pour la fabrication d'un médicament pour le traitement ou la prévention d'une co-infection provoquée par un procaryote intracellulaire obligé, un virus à ADN ou un rétrovirus chez un individu mammifère, infecté par le VIH, dans laquelle ledit médicament est adapté pour administrer une quantité thérapeutiquement efficace de gallium à l'individu mammifère, et dans laquelle la quantité thérapeutiquement efficace est telle qu'une concentration en gallium dans le plasma sanguin est fournie qui est suffisante pour permettre le traitement ou la prévention de l'infection et de la co-infection.
